(19) **Europäisches Patentamt**
**European Patent Office**
**Office européen des brevets**

(11) **EP 2 747 694 B1**

(12) **EUROPEAN PATENT SPECIFICATION**

(45) Date of publication and mention
of the grant of the patent:
**04.05.2016 Bulletin 2016/18**

(21) Application number: **11746584.9**

(22) Date of filing: **26.08.2011**

(51) Int Cl.:
**A61B 90/00** (2016.01)    **A61B 34/20** (2016.01)

(86) International application number:
**PCT/EP2011/064689**

(87) International publication number:
**WO 2013/029644 (07.03.2013 Gazette 2013/10)**

(54) **METHOD OR DETERMINING THE SHAPE OF A SURGICAL INSTRUMENT AND SURGICAL INSTRUMENT HAVING A DEFORMABLE BODY**

VERFAHREN ZUR BESTIMMUNG DER FORM EINES CHIRURGISCHEN INSTRUMENTES SOWIE CHIRURGISCHES INSTRUMENT MIT VERFORMBAREM KÖRPER

PROCÉDÉ DE DÉTERMINATION DE LA FORME D'UN INSTRUMENT CHIRURGICAL ET INSTRUMENT CHIRURGICAL AYANT UN CORPS DÉFORMABLE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**02.07.2014 Bulletin 2014/27**

(73) Proprietor: **Brainlab AG**
**85622 Feldkirchen (DE)**

(72) Inventors:
• **BIRKENBACH, Rainer**
  **85435 Erding (DE)**

• **MANUS, Johannes**
  **81479 Munich (DE)**

(74) Representative: **Schwabe - Sandmair - Marx**
**Patentanwälte Rechtsanwalt**
**Partnerschaft mbB**
**Stuntzstraße 16**
**81677 München (DE)**

(56) References cited:
**DE-A1-102009 021 705    US-A1- 2003 229 279**
**US-A1- 2007 106 114    US-A1- 2008 139 916**
**US-A1- 2008 171 934**

EP 2 747 694 B1

**Description**

The present invention relates to a method which allows the shape of a surgical instrument having a deformable body and at least one tracking sensor to be determined. The present invention also relates to a corresponding surgical instrument.

In the field of surgical tracking technology, electromagnetic (EM) tracking technology provides the ability to track very tiny sensors which can be integrated in medical instruments. Thus, even very small instruments such as for example catheters can be tracked inside a patient's body into which they have been introduced. However, the number of sensors which can be placed in the instrument is very limited. The number of channels an EM tracking system can track simultaneously is restricted, as is the space available for placing the sensors and their cabling in the instrument. For these reasons, it is typically the case that only a few parts of the instrument to be tracked are provided with a tracking sensor. One tracking sensor is for example placed in the tip of a catheter, such that only the position of the tip of the catheter can be tracked. The shape of the catheter remains undisclosed to the user.

US 2008/171934 discloses a method for determining the spatial position and the shape of a surgical instrument having a deformable body, comprising the steps of: providing bending or flexure properties of the surgical instrument; defining at least one parameter which influences the shape of the instrument; determining the spatial position and/or orientation of at least one tracking sensor of the surgical instrument; determining the value of the at least one parameter by means of a second tracking sensor; and calculating the shape of at least one part of the surgical instrument with the aid of the bending or flexure properties together with the determined value of the at least one parameter and the determined spatial position and/or orientation of the at least one tracking sensor.

It is an object of the present invention to provide a solution to the aforementioned problems. In particular, the present invention aims to enable the shape of an instrument, in particular the overall shape of an instrument having a limited number of tracking sensors, in particular only one tracking sensor, to be determined. It is another object of the present invention to provide a corresponding instrument having a limited number of tracking sensors, in particular only one tracking sensor, wherein the shape of at least part of the instrument but in particular the overall shape of the instrument can be determined by tracking the available tracking sensors.

The above objects are achieved by a method according to claim 1 and by a surgical instrument according to claim 11. The sub-claims define advantageous embodiments of this method and instrument, respectively.

In accordance with the present invention, a method is provided for determining the shape of a surgical instrument having a deformable body.

In other words, it is necessary to know how the instrument deforms under the influence of certain parameters such as for example force, pressure, stress or the like. Parameters need to be defined which influence the shape of the instrument. For example, a force acting transversely on the tip of a longitudinal end will cause the instrument body to bend. The greater the force, the greater the deflection. Therefore, the value of the defined parameters also has to be known. Once the nature and magnitude of deformation in the shape of the instrument as a function of certain parameters and their value has been ascertained, the actual shape of the instrument can be calculated from the elasticity model of the surgical instrument and the parameters, and their value, acting on the surgical instrument.

The expression "deformable body" as used herein is to be understood as describing a body which is deformable, irrespective of whether the body, once deformed, will return to its original shape automatically (elastic body) or will keep its shape, providing no further influence acts on it. In other words, the deformable body can act as a resilient body, such as for example a spring, or a ductile body, such as for example a modelling material (plastic body). Moreover, the body can be an elastic body or a plastic body.

By determining the spatial position and/or orientation of at least one tracking sensor of the surgical instrument, the spatial position and/or orientation of at least one point of the surgical instrument is known.

Since the spatial position and/or orientation of at least one point of the surgical instrument is known, and the shape of the instrument is known, the position and/or orientation of at least one part of the surgical instrument can be calculated with the aid of the elasticity model of the instrument, by inputting the values for the spatial position and/or orientation of the at least one tracking sensor and the determined value of the at least one parameter into the elasticity model.

For example, the shape of the instrument can be described by a simple parameterised function dependent on a number of parameters $a_j$, wherein every point on the instrument $x_i^0$ can be calculated by the function

$$x_i = f(i; a_1, \ldots, a_n).$$

If m tracking sensors are placed in the instrument, the position and/or orientation of m points of the instrument are known by determining the position and/or orientation of said m sensors. Consequently, a system of m equations can be established:

$$x_1 = f(1; a_1, \ldots, a_n)$$

$$x_m = f(m; a_1, \ldots, a_n).$$

This system of equations can then be solved for the parameter $a_j$ using a standard equation solver. The position of every point of the instrument can then be calculated using the function together with the known parameters $a_j$.

The above embodiment can be applied to instruments which exhibit simple geometries, for example a catheter comprising a tubular body.

More complex instruments may not be able to be described by a simple function dependent on a small number of parameters. In this case, a finite elements algorithm may be applied. The instrument can be modelled on the basis of its physical properties, and a finite elements algorithm can be used to calculate the shape of the instrument, such that the position of every point of the instrument is known. Additionally, the determined position and/or orientation of the sensors can be used to determine the correct boundary conditions for the finite elements calculation.

In accordance with a preferred embodiment of the present invention, the tracking sensor is an electromagnetic (EM) tracking sensor, wherein the position and/or orientation of the tracking sensor is determined by an electromagnetic (EM) tracking method.

The value of the at least one predetermined parameter is determined by means of a sensor, in particular a sensor which is placed in the surgical instrument. Such a sensor may be configured to measure a distance, force, pressure and/or stress or any other suitable parameter which can cause the surgical instrument to be deformed.

In accordance with another embodiment, the spatial position and the shape of an instrument having only one EM sensor is determined, wherein the EM sensor is placed at the tip of the instrument, and the additional information of the instrument is obtained from another sensor which is not a tracking sensor. Sensors for measuring a force acting on the instrument and/or bending/stress sensors can for example be provided, or even a sensor which measures the length of a pull-wire of a catheter which is pulled in order to change the shape of the catheter.

Generally, any sensor which provides information for calculating the shape of the surgical instrument can be used.

The method of the present invention can also be used to calculate the shape of two-dimensional structures such as electrode grid sheets used for cortical stimulation and mapping. The position of the electrodes could be calculated by assimilating the shape of the electrode grid sheet as determined on the basis of the measurements of a number of tracking sensors attached to the sheet.

It is also possible to determine the value of more parameters than is necessary for calculating the shape of the instrument, in order to provide a reliability measurement, such that distortions in the EM tracking system and potential tracking inaccuracies can be detected. Field distortions caused by ferrous or conductive materials can therefore be detected and the user warned.

In accordance with another preferred embodiment of the present invention, not only the position and/or orientation of at least one part of the surgical instrument but rather the overall shape of the instrument is calculated. This allows the overall shape of the instrument to be displayed in a user-friendly manner within a medical navigation method. The user can then see the whole instrument in its actual shape in relation to other surgical data such as in particular images of the patient.

Another aspect of the present invention relates to a program which, when running on a computer or loaded onto a computer, causes the computer to perform a method as described above. Another aspect of the present invention relates to a program storage medium in which the above program is stored.

Another aspect of the present invention relates to a surgical instrument which has a deformable body and at least two sensors, wherein at least one sensor is a tracking sensor and at least one other sensor is a sensor which provides additional information for calculating the shape of at least one part of the surgical instrument, namely by determining the value of a predetermined parameter which influences the shape of the instrument. The at least one other sensor can be configured to measure a distance, force, pressure, stress or any other parameter which influences the shape of the surgical instrument.

An embodiment not of the present invention is described below by referring to the enclosed drawing.

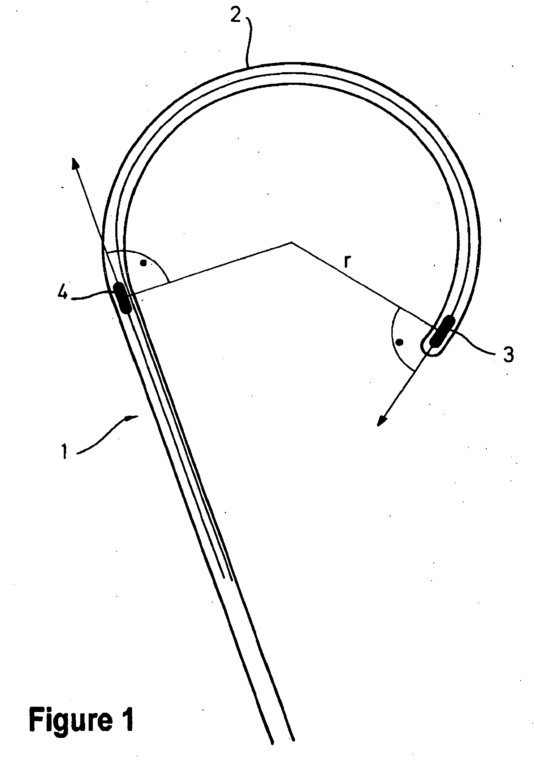

Figure 1 shows an elongated catheter 1 having a body 2 with a lower, rigid section and an upper, deformable (for example an elastic or a plastic) section. Two sensors 3, 4 are integrated in the catheter 1 and/or catheter body 2, wherein the sensor 3 is an EM tracking sensor placed at the tip of the catheter and the sensor 4 is also an EM tracking sensor. The spatial position and orientation of both sensors 3 and 4 can be determined by means of an EM tracking system. Arrows extending from the sensors 3 and 4 indicate the tangential direction of the catheter body 2 and the position of the sensors 3 and 4.

Since the material properties do not change in the deformable section between the sensors 3 and 4, pulling a pull-wire attached to the tip of the catheter out of the proximal end of the catheter will cause a uniform deformation of the catheter body 2 between the sensors 3 and 4.

Since the material properties of the deformable body section are known, an elasticity model of the instrument can be provided, wherein the shape of the deformable body section can be calculated with the aid of the elasticity model, and the spatial position and orientation of the catheter parts in which the sensors 3 and 4 are placed can be calculated from the measured position and orientation of the sensors 3 and 4 by means of an EM tracking system.

The overall shape of the catheter 1 can thus be calculated using only two EM sensors.

However in accordance with the invention, the EM tracking sensor 4 could also be replaced with a sensor which is not an EM tracking sensor. For example, a sensor could be provided which measures the amount of pull-wire which is pulled out of the proximal end of the catheter 1, so as to obtain information on the extent to which the deformable part of the catheter body 2 will deform. Such a sensor could also be provided outside of the catheter body 2, such that the catheter 1 only comprises one EM tracking sensor and can therefore be designed even smaller.

**Claims**

1. A method for determining the spatial position and the shape of an elongated surgical instrument (1) having a deformable body (2), said method comprising the steps of:

   - providing an elasticity model of the surgical instrument (1);
   - defining at least one parameter which influences the shape of the instrument (1);
   - determining the spatial position and orientation of the tip of the surgical instrument (1) by means of a tracking sensor (3) placed at the tip of the instrument (1);
   - determining the value of the at least one parameter by means of a sensor (4) which is not a tracking sensor;

   calculating the shape of at least one part of the surgical instrument (1) with the aid of the elasticity model together with the determined value of the at least one parameter and the determined spatial position and orientation of the at least one tracking sensor (3).

2. The method according to claim 1, wherein the tracking sensor (3) is an EM tracking sensor and its position and/or orientation is determined by an EM tracking method.

3. The method according to any one of claims 1 or 2, wherein the value of the at least one predetermined parameter, in particular a distance, force, pressure and/or stress, is determined by means of said sensor (4).

4. The method according to any one of claims 1 to 3, wherein the elasticity model is over-determined by inputting the determined value of the at least one predetermined parameter and the determined spatial position and/or orientation of the at least one tracking sensor (3).

5. The method according to any one of claims 1 to 4, wherein the overall shape of the instrument (1) is calculated.

6. The method according to any one of claims 1 to 5, wherein a finite elements method is used to calculate the position and/or orientation of at least one part of the surgical instrument (1), in particular the overall shape of the instrument (1).

7. The method according to any one of claims 1 to 6, wherein at least part of the instrument (1), in particular the overall shape of the instrument (1), is displayed within a medical navigation method.

8. A program which, when running on a computer or when loaded onto a computer, causes the computer to perform a method according to any one of the preceding claims.

9. A program storage medium in which the program according to the preceding claim is stored.

10. An elongated surgical instrument (1) having a deformable body (2) comprising at least two sensors (3, 4), wherein at least one sensor is a tracking sensor (3) placed at the tip of the instrument (1) by means of which the spatial position and orientation of the tip of the surgical instrument (1) can be determined and at least one other sensor is a sensor (4) which is not a tracking sensor, by means of which the value of a predetermined parameter which influences the shape of the instrument (1) can be determined.

11. The surgical instrument according to claim 10, wherein the tracking sensor (3) is an EM tracking sensor and/or the at least one other sensor (4) is a sensor which is configured to measure a distance, force, pressure or stress.

**Patentansprüche**

1. Verfahren zum Bestimmen der räumlichen Position und der Form eines gestreckten chirurgischen Instruments (1) mit einem verformbaren Körper (2), wobei das Verfahren folgende Schritte umfasst:

   - Bereitstellen eines Elastizitätsmodells des chirurgischen Instruments (1);
   - Definieren zumindest eines Parameters, welcher die Form des Instruments (1) beeinflusst;
   - Bestimmen der räumlichen Lage und Ausrichtung der Spitze des chirurgischen Instruments (1) mittels eines an der Spitze des Instruments (1) platzierten Trackingsensors (3);
- Bestimmen der Größe des zumindest einen Parameters mittels eines Sensors (4), welcher kein Trackingsensor ist;
- Berechnen der Form zumindest eines Teils des chirurgischen Instruments (1) mithilfe des Elastizitätsmodels zusammen mit der bestimmten Größe des zumindest einen Parameters und der bestimmten räumlichen Lage und Ausrichtung des zumindest einen Trackingsensors (3).

2. Verfahren gemäß Anspruch 1, wobei der Trackingsensor (3) ein EM-Träckingsensor ist und seine Lage und/oder Ausrichtung mittels eines EM-Tracking-Verfahrens bestimmt wird.

3. Verfahren gemäß einem der Ansprüche 1 oder 2, wobei die Größe des zumindest einen vordefinierten Parameters, insbesondere ein Abstand, eine Kraft, ein Druck und/oder eine Spannung, mittels des Sensors (4) bestimmt wird.

4. Verfahren gemäß einem der Ansprüche 1 bis 3, wobei das Elastizitätsmodell durch Eingeben der bestimmten Größe des zumindest einem vorbestimmten Parameters und der bestimmten räumlichen Lage und/oder Ausrichtung des zumindest einen Trackingsensors (3) überbestimmt ist.

5. Verfahren gemäß einem der Ansprüche 1 bis 4, wobei die gesamte Form des Instruments (1) berechnet wird.

6. Verfahren gemäß einem der Ansprüche 1 bis 5, wobei ein Finite-Elemente-Verfahren zur Berechnung der Lage und/oder Ausrichtung zumindest eines Teils des chirurgischen Instruments (1), insbesondere der gesamten Form des Instruments (1) verwendet wird.

7. Verfahren gemäß einem der Ansprüche 1 bis 6, wobei zumindest ein Teil des Instruments (1) insbesondere die gesamte Form des Instruments (1) innerhalb eines medizinischen Navigationsverfahrens dargestellt wird.

8. Programm, welches, wenn es auf einem Computer läuft oder wenn es auf einem Computer geladen ist, den Computer dazu veranlasst, ein Verfahren gemäß einem der voranstehenden Ansprüche durchzuführen.

9. Programm-Speichermedium, auf welchem das Programm gemäß dem voranstehenden Anspruch gespeichert ist.

10. Gestrecktes chirurgisches Instrument (1) mit einem verformbaren Körper (2) umfassend zumindest zwei Sensoren (3, 4), wobei zumindest ein Sensor ein Trackingsensor (3) ist, der an der Spitze des Instruments (1) platziert ist und mittels welchem die räumliche Lage und Ausrichtung der Spitze des chirurgischen Instruments (1) bestimmt werden kann, und zumindest ein weitere Sensor ein Sensor (4) ist, welcher kein Trackingsensor ist und mittels welchem die Größe eines vorbestimmten Parameters bestimmt werden kann, welcher die Form des Instruments (1) beeinflusst.

11. Chirurgisches Instrument gemäß Anspruch 10, wobei der Trackingsensor (3) ein EM-Trackingsensor ist und/oder der zumindest eine weitere Sensor (4) ein Sensor ist, welcher dazu ausgestaltet ist, einen Abstand, eine Kraft, einen Druck, oder eine Spannung zu messen.

**Revendications**

1. Procédé de détermination de la position spatiale et de la forme d'un instrument chirurgical (1) allongé ayant un corps déformable (2), ledit procédé comprenant les étapes consistant à :

   - fournir un modèle d'élasticité de l'instrument chirurgical (1) ;
   - définir au moins un paramètre qui influence la forme de l'instrument (1) ;
   - déterminer la position spatiale et l'orientation de la pointe de l'instrument chirurgical (1) au moyen d'un capteur de suivi (3) placé au niveau de la pointe de l'instrument (1) ;
   - déterminer la valeur de l'au moins un paramètre au moyen d'un capteur (4) qui n'est pas un capteur de suivi ;

   calculer la forme d'au moins une partie de l'instrument chirurgical (1) à l'aide du modèle d'élasticité conjointement avec la valeur déterminée de l'au moins un paramètre et la position et l'orientation spatiales déterminées de l'au moins un capteur de suivi (3).

2. Procédé selon la revendication 1, où le capteur de suivi (3) est un capteur de suivi EM et sa position et/ou orientation sont déterminées par une procédé de suivi EM.

3. Procédé selon l'une quelconque des revendications 1 ou 2, où la valeur de l'au moins un paramètre prédéterminé, en particulier une distance, une force, une pression et/ou une contrainte, est déterminée au moyen dudit capteur (4).

4. Procédé selon l'une quelconque des revendications 1 à 3, où le modèle d'élasticité est surdéterminé en saisissant la valeur déterminée de l'au moins un paramètre prédéterminé et la position et/ou orientation spatiales déterminées de l'au moins un capteur de suivi (3).

5. Procédé selon l'une quelconque des revendications 1 à 4, où la forme générale de l'instrument (1) est calculée.

6. Procédé selon l'une quelconque des revendications 1 à 5, où une méthode des éléments finis est utilisée pour calculer la position et/ou l'orientation d'au moins une partie de l'instrument chirurgical (1), en particulier la forme générale de l'instrument (1).

7. Procédé selon l'une quelconque des revendications 1 à 6, où au moins une partie de l'instrument (1), en particulier la forme générale de l'instrument (1) est présentée dans un procédé de navigation médicale.

8. Programme qui, lorsqu'il est exécuté sur un ordinateur ou chargé dans un ordinateur, déclenche l'exécution par l'ordinateur d'un procédé selon l'une quelconque des revendications précédentes.

9. Support de stockage de programme sur lequel le programme selon la revendication précédente est stocké.

10. Instrument chirurgical allongé (1) ayant un corps déformable (2) comportant au moins deux capteurs (3, 4), où au moins un capteur est un capteur de suivi (3) placé au niveau de la pointe de l'instrument (1) au moyen duquel la position et l'orientation spatiales de la pointe de l'instrument chirurgical (1) peuvent être déterminée et où au moins un autre capteur est un capteur (4) qui n'est pas un capteur de suivi, au moyen duquel la valeur d'un paramètre prédéterminé ayant une influence sur la forme de l'instrument (1) peut être déterminée.

11. Instrument chirurgical selon la revendication 10, où le capteur de suivi (3) est un capteur de suivi EM et/ou l'au moins un autre capteur (4) est une capteur conçu pour mesurer une distance, une force, une pression ou une contrainte.

**Figure 1**

**EP 2 747 694 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2008171934 A